# EUROPÄISCHE PATENTSCHRIFT

(11) **EP 1 227 811 B1**
(45) Veröffentlichungstag und Bekanntmachung des Hinweises auf die Patenterteilung: **14.01.2004**
(21) Anmeldenummer: 00972764.5
(22) Anmeldetag: 14.10.2000
(51) Int. Cl.: A61K 31/415, A61P 9/10

(54) **VERWENDUNG VON 2-IMIDAZOLYL-SUBSTITUIERTEN CARBINOLEN ZUR HERSTELLUNG EINES MEDIKAMENTS ZUR BEHANDLUNG ODER PROPHYLAXE VON DURCH ISCHÄMISCHEN ZUSTÄNDEN BEWIRKTEN KRANKHEITEN**
USE OF 2-IMIDAZOLYL SUBSTITUTED CARBINOLS FOR PRODUCTION OF A MEDICAMENT FOR TREATMENT OR PROPHYLAXIS OF DISEASE STATES AS A RESULT OF ISCHAEMIC CONDITIONS
UTILISATION DE CARBINOLS SUBSTITUES PAR 2-IMIDAZOLYLE POUR PRODUIRE UN MEDICAMENT ASSURANT LE TRAITEMENT ET LA PROPHYLAXIE D'AFFECTIONS INDUITES PAR DES ETATS ISCHEMIQUES

(30) Priorität: 27.10.1999 DE 19951701
(43) Veröffentlichungstag der Anmeldung: 07.08.2002
(73) Patentinhaber: Aventis Pharma Deutschland GmbH, 65929 Frankfurt am Main (DE)
(72) Erfinder: WEICHERT, Andreas, 22391 Hamburg (DE); ALBUS, Udo, 61197 Florstadt (DE); JANSEN, Hans-Willi, 65527 Niedernhausen (DE)
(86) Internationale Anmeldenummer: PCT/EP2000/010126
(87) Internationale Veröffentlichungsnummer: WO 2001/030327

(56) Entgegenhaltungen:
- GB-A- 1 363 382
- US-A- 4 213 994
- US-A- 5 063 220
- OTA, SHUNSAKU ET AL: "Synthesis and application of imidazole derivatives. Synthesis of (1-methyl-1H-imidazol-2-yl)methanol derivatives and conversion into carbonyl compounds" CHEM. PHARM. BULL. (1987), 35(3), 1058-69 , XP001015963
- VAN DER STELT, CORNELIS ET AL: "Synthesis and pharmacological properties of a series of.alpha.,.alpha.-diaryl-1H-imidazole-2-me thanol derivatives" EUR. J. MED. CHEM. - CHIM. THER. (1978), 13(3), 251-8 , XP002175827

## Beschreibung

Kürzlich sind Patentanmeldungen publiziert worden, die Verbindungen mit der Formel I beanspruchen: 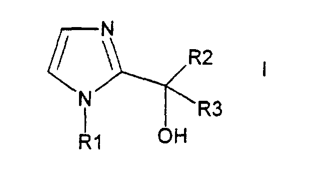 In der DE-OS 23 05 212 sind Verbindungen ähnlicher Konstitution mit analgetischer, anorektischer, antiphlogistischer und antipyretischer Aktivität beschrieben. In DE-OS 2164919 wird die anticholesterämische Wirkung dieser Verbindungen beansprucht. in WO 97 49 704 sind Vertreter dieser Verbindungsklasse in der Indikation Krebskrankheiten beschrieben, wobei sie in den Vitamin A-Säurestoffwechset eingreifen. In JP 63270665 wird deren Antiulcus-Aktivität beschrieben.

In keiner dieser Veröffentlichungen findet sich ein Hinweis auf eine Wirkung dieser Verbindungen bei ischämischen Zuständen.

Die Erfindung betrifft die Verwendung von 2-Imidazol-substituierte Carbinolen I und von deren pharmazeutisch verträglichen Salzen
worin bedeuten:
- R1: geradkettiges oder verzweigtes C₁-C₈- Alkyl oder Phenyl-(CH₂)ₘ-;
m Null, 1 oder 2;
wobei der Phenylkern unsubstituiert ist oder einen bis drei Substituenten aus den Gruppen F, Cl, CH₃ oder CH₃O trägt,
- R2 und R3: geradkettiges oder verzweigtes C₁-C₆-Alkyl oder Phenyl,
wobei der Phenylkern unsubstituiert ist oder einen bis drei Substituenten aus den Gruppen F, Cl, CH₃ oder CH₃O trägt;
oder
- R2 und R3: gemeinsam einen (C₅-C₆)- Ring bilden können,
der unsubstituiert ist oder an den Phenylringe anneliert sind für die Herstellung eines Medikaments zur Therapie oder Prophylaxe von ischämischen Zuständen.

Bevorzugt verwendet werden Verbindungen I, in denen bedeuten:
- R1: geradkettiges oder verzweigtes C₄ C₆- Alkyl, Phenyl oder Benzyl,
wobei der Phenylkern unsubstituiert ist oder einen bis drei Substituenten aus den Gruppen F, Cl, CH₃ oder CH₃O trägt;
- R2 und R3: geradkettiges oder verzweigtes C₁-C₆- Alkyl oder Phenyl,
wobei der Phenylkern unsubstituiert ist oder einen bis drei Substituenten aus den Gruppen F, Cl, CH₃ oder CH₃O trägt;
oder
- R2 und R3: gemeinsam mit dem C-Atom, an das sie gebunden sind, ein Fluoren bilden.

Enthält einer der drei Substituenten R1, R2 oder R3 ein Asymmetriezentrum, so gehören sowohl S als auch R konfigurierte Verbindungen zur Erfindung. Die erfindungsgemäß verwendeten Verbindungen können als optische isomere, als Diastereoisomere, als Racemate oder als Gemische derselben vorliegen.

Überraschenderweise zeichnen sich diese bereits bekannten Verbindungen durch eine Inhibition des Na⁺/H⁺-Austauschs aus. Somit sind sie infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die Verbindungen der Formel 1 infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z. B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die erfindungsgemäß verwendeten Verbindungen auch als Arzneimittel zur Behandlung von Ischämien des Nervensystems, insbesondere des Zentralnervensystems, geeignet, wobei sie z. B. zur Behandlung des Schlaganfalls oder des Hirnödems geeignet sind. Darüber hinaus eignen sich die Verbindungen der Formel I ebenfalls zur Behandlungen von Formen des Schocks, wie beispieiweise des allergischen, cardiogenen, hypovolämischen und des bakteriellen Schocks.

Die erfindungsgemäß verwendeten Verbindungen sind wirkungsvolle Inhibitoren des zellulären Natrium-Protonen-Antiporters (Na⁺/H⁺ -Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, usw.. Darüber hinaus sind die Verbindungen der Formel 1 für die präventive Therapie zur Verhinderung der Genese des Bluthochdrucks, beispielweise der essentiellen Hypertonie, geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen I mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen I, gewünschtenfalls mit den dafür üblichen Substanzen wie Lösungsvermittlern, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des erfindungsgemäß verwendeten Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

### Experimenteller Teil

### Liste der Abkürzungen:

- RT: Raumtemperatur
- EE: Ethylacetat (EtOAc)
- Smp: Schmelzpunkt
- THF: Tetrahydrofuran
- eq.: Äquivalent

### Beispiel 1: 9-(1-Benzyl-1H-imidazol-2-yl)-9H-fluoren-9-ol, farbloser Feststoff, Smp. 149°C, M⁺+H= 339.

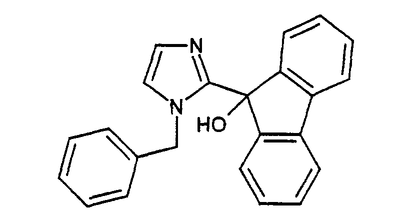

Zu N-Benzyl-imidazol in THF wird bei -70°C 1.2 eq n-Butyllithium addiert. Innerhalb einer Stunde wird auf -20°C erwärmen gelassen und dann erneut auf -70°C gekühlt. Nach Zugabe von 1 eq Fluorenon in THF wird innerhalb 5 h auf RT erwärmen gelassen. Wäßrige Aufarbeitung, Extraktion mit EE, gefolgt von anschließender Trocknung der organischen Phasen über Magnesiumsulfat und Evaporation der Lösungsmittel liefert einen festen, gelblichen Rückstand. Verreiben mit Diethylether liefert einen Feststoff, der abgesaugt wird.

### Beispiel 2: (1-Butyl-1H-imidazol-2-yl)-phenyl-4-fluorphenyl-carbinol, farbloser Feststoff, Smp. 138°C, M⁺+H= 325.

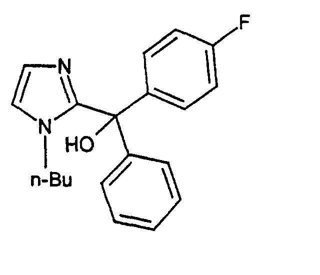

Durchführung wie unter 1) beschrieben, lediglich unter Einsatz von N-n-Butylimidazol und 4-Fluorphenyl-phenyl-keton.

### Pharmakologische Daten:

### Inhibition des Na⁺/H⁺-Exchangers von Kaninchenerythrocyten

Weiße Neuseeland-Kaninchen (Ivanovas) erhielten eine Standard-Diät mit 2% Cholesterin für sechs Wochen, um den Na⁺IH⁺-Austausch zu aktivieren und so den Na⁺-Influx in die Erythrocyten via Na⁺/H⁺-Austausch flammenphotometrisch bestimmen zu können. Das Blut wurde den Ohrarterien entnommen und durch 25 IE Kalium-Heparin ungerinnbar gemacht. Ein Teil jeder Probe wurde zur Doppelbestimmung des Hämatokrits durch Zentrifugieren benutzt. Aliquots von jeweils 100 µl dienten zur Messung des Na⁺-Ausgangsgehalts der Erythrocyten.

Um den Amilorid-sensitiven Natrium-Influx zu bestimmen, wurden 100 µl jeder Blutprobe in jeweils 5 ml eines hyperosmolaren Salz-Sucrose-Mediums (mmol/l: 140 NaCl, 3 KCl, 150 Sucrose, 0,1 Ouabain, 20 Tris-hydroxymethyl-aminomethan) bei pH 7,4 und 37°C inkubiert. Die Erythrocyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺-Nettoinflux wurde aus der Differenz zwischen Natrium-Ausgangswerten und dem Natriumgehalt der Erythrocyten nach inkubation errechnet. Der Amiloridhemmbare Natrium-Influx ergab sich aus der Differenz des Natriumgehalts der Erythrocyten nach Inkubation mit und ohne Amilorid 3 x 10⁻⁴ mol/l. Auf diese Weise wurde auch bei den erfindungsgemäßen Verbindungen verfahren.

### Ergebnisse

| Inhibition des Na⁺/H⁺-Exchangers: | |
|---|---|
| Beispiel | IC₅₀(µmol / l) |
| 1: | 9.3 |
| | |
| 2: | < 50 |

## Patentansprüche

1. Verwendung einer Verbindung I, 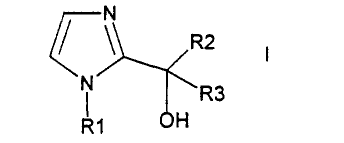 worin bedeuten:
R1 geradkettiges oder verzweigtes C₁-C₈- Alkyl oder Phenyl-(CH₂)ₘ-;
m Null, 1 oder 2;
wobei der Phenylkern unsubstituiert ist oder einen bis drei Substituenten aus den Gruppen F, Cl, CH₃ oder CH₃O trägt,
R2 und R3 geradkettiges oder verzweigtes C₁-C₆- Alkyl oder Phenyl,
wobei der Phenylkern unsubstituiert ist oder einen bis drei Substituenten aus den Gruppen F, Cl, CH₃ oder CH₃O trägt;
oder
R2 und R3 gemeinsam einen (C₅-C₆)- Ring bilden können,
der unsubstituiert ist oder an den Phenylringe anneliert sind, und von deren pharmazeutisch verträglichen Salzen zur Herstellung eines Medikaments zur Behandlung oder Prophylaxe von durch ischämische Zustände bewirkten Krankheiten.

2. Verwendung einer Verbindung I nach Anspruch 1 zur Herstellung eines Medikaments zur Behandlung oder Prophylaxe des Herzinfarkts.

3. Verwendung einer Verbindung I nach Anspruch 1 zur Herstellung eines Medikaments zur Behandlung oder Prophylaxe der Angina Pectoris.

4. Verwendung einer Verbindung I nach Anspruch 1 zur Herstellung eines Medikaments zur Behandlung oder Prophylaxe von ischämischen Zuständen des Herzens.

5. Verwendung einer Verbindung 1 nach Anspruch 1 zur Herstellung eines Medikaments zur Behandlung oder Prophylaxe von ischämischen Zuständen des peripheren und zentralen Nervensystems und des Schlaganfalls.

6. Verwendung einer Verbindung I nach Anspruch 1 zur Herstellung eines Medikaments zur Behandlung oder Prophylaxe von ischämischen Zuständen peripherer Organe und Gliedmaßen.

7. Verwendung einer Verbindung I nach Anspruch 1 zur Herstellung eines Medikaments zur Behandlung von Schockzuständen.

8. Verwendung einer Verbindung I nach Anspruch 1 zur Herstellung eines Medikaments zum Einsatz bei chirurgischen Operationen und Organtransplantationen.

9. Verwendung einer Verbindung I nach Anspruch 1 zur Herstellung eines Medikaments zur Konservierung und Lagerung von Transplantaten für chirurgische Maßnahmen.

## Claims

1. The use of a compound I 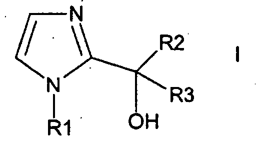 in which:
R1 is straight-chain or branched C₁-C₈-alkyl or phenyl-(CH₂)ₘ-;
m is zero, 1 or 2;
where the phenyl nucleus is unsubstituted or carries one to three substituents from the groups F, Cl, CH₃ or CH₃O,
R2 and R3 are straight-chain or branched C₁-C₆-alkyl or phenyl,
where the phenyl nucleus is unsubstituted or carries one to three substituents from the groups F, Cl, CH₃ or CH₃O;
or
R2 and R3 can together form a (C₅-C₆) ring,
which is unsubstituted or to which phenyl rings are fused, and of its pharmaceutically tolerable salts for the production of a medicament for the treatment or prophylaxis of diseases caused by ischemic conditions.

2. The use of a compound I as claimed in claim 1 for the production of a medicament for the treatment or prophylaxis of cardiac infarct.

3. The use of a compound I as claimed in claim 1 for the production of a medicament for the treatment or prophylaxis of angina pectoris.

4. The use of a compound I as claimed in claim 1 for the production of a medicament for the treatment or prophylaxis of ischemic conditions of the heart.

5. The use of a compound I as claimed in claim 1 for the production of a medicament for the treatment or prophylaxis of ischemic conditions of the peripheral and central nervous system and of stroke.

6. The use of a compound I as claimed in claim 1 for the production of a medicament for the treatment or prophylaxis of ischemic conditions of peripheral organs and limbs.

7. The use of a compound I as claimed in claim 1 for the production of a medicament for the treatment of states of shock.

8. The use of a compound I as claimed in claim 1 for the production of a medicament for use in surgical operations and organ transplantation.

9. The use of a compound I as claimed in claim 1 for the production of a medicament for the preservation and storage of transplants for surgical measures.

## Revendications

1. Utilisation d'un composé I : 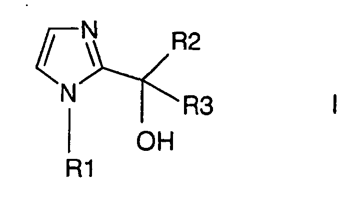 où :
R1 représente alkyle en C₁-C₈ linéaire ou ramifié ou phényle-(CH₂)ₘ- ;
m représente 0, 1 ou 2 ;
où le noyau phényle est non substitué ou porte 1 à 3 substituants des groupes F, Cl, CH₃ ou CH₃O,
R2 et R3 représentent alkyle en C₁-C₆ linéaire ou ramifié ou phényle,
où le noyau phényle est non substitué ou porte 1 à 3 substituants des groupes F, Cl, CH₃ ou CH₃O ; ou bien
R2 et R3 peuvent former ensemble un cycle en (C₅-C₆),
qui est non substitué ou auquel sont condensés des cycles phényle,
et de ses sels pharmaceutiquement acceptables pour la production d'un médicament pour le traitement ou la prophylaxie de maladies dues à des états ischémiques.

2. Utilisation d'un composé I selon la revendication 1 pour la production d'un médicament pour le traitement ou la prophylaxie de l'infarctus cardiaque.

3. Utilisation d'un composé I selon la revendication 1 pour la production d'un médicament pour le traitement ou la prophylaxie de l'angine de poitrine.

4. Utilisation d'un composé I selon la revendication 1 pour la production d'un médicament pour le traitement ou la prophylaxie d'états ischémiques du coeur.

5. Utilisation d'un composé I selon la revendication 1 pour la production d'un médicament pour le traitement ou la prophylaxie d'états ischémiques du système nerveux périphérique et central et de l'apoplexie cérébrale.

6. Utilisation d'un composé I selon la revendication 1 pour la production d'un médicament pour le traitement ou la prophylaxie des états ischémiques d'organes périphériques et de membres.

7. Utilisation d'un composé I selon la revendication 1 pour la production d'un médicament pour le traitement des états de choc.

8. Utilisation d'un composé I selon la revendication 1 pour la production d'un médicament destiné à être utilisé lors des opérations chirurgicales et des transplantations d'organes.

9. Utilisation d'un composé I selon la revendication 1 pour la production d'un médicament pour la conservation et le stockage de greffes pour des mesures chirurgicales.
